# EUROPEAN PATENT APPLICATION

(11) **EP 4 342 361 A1**
(43) Date of publication of application: **27.03.2024**
(21) Application number: 22804496.2
(22) Date of filing: 20.04.2022
(51) Int. Cl.: A61B 3/135

(54) **OPHTHALMIC MICROSCOPE**

(30) Priority: 19.05.2021 JP 2021084440
(71) Applicant: TOPCON CORPORATION, Tokyo 174-8580 (JP)
(72) Inventor: MIZUNO, Akira, Tokyo 174-8580 (JP); MIYASHITA, Kenji, Tokyo 174-8580 (JP)
(74) Representative: Schmitt-Nilson Schraud Waibel Wohlfrom Patentanwälte Partnerschaft mbB
(86) International application number: PCT/JP2022/018287
(87) International publication number: WO 2022/244582

(57) **Abstract**

An ophthalmic microscope capable of acquiring an observation image that meets observation target and purpose of an eye to be examined and an examiner's dominant eye is provided. The ophthalmic microscope includes an observation system configured to observe an eye to be examined, the observation system including an objective lens and a pair of observation optical paths, an imaging system capable of stereoscopically imaging respective observation lights of the eye to be examined from the observation optical paths, and a mode switching unit capable of selectively switching among a first mode for acquiring a first image obtained by stereoscopically imaging the observation lights from both the observation optical paths using the imaging system, a second mode for acquiring only a second image obtained by imaging the observation light from one of the observation optical paths using the imaging system, and a third mode for acquiring only a third image obtained by imaging the observation light from the other of the observation optical paths using the imaging system.

## Description

### Technical Field

The presently disclosed subject matter relates to an ophthalmic microscope for use in observation of an eye to be examined.

### Background Art

A slit lamp microscope (also called a slit lamp or a slit lamp microscope) is known as an ophthalmic microscope to be used for observing an eye to be examined. This slit lamp microscope uses slit light to pick up an optical slice of a region of interest of an eye to be examined, thereby observing a cross-section of the region of interest and acquiring an image of this cross-section.

The slit lamp microscope includes a lighting system and an observation system. The lighting system irradiates an eye to be examined with width-adjusted slit light. The observation system leads return light from the eye to be examined irradiated with the slit light from an objective lens through a pair of left and right observation optical paths to a pair of left and right eyepieces, which makes it possible for an examiner to observe the eye to be examined through the eyepieces.

At this time, a work of gazing through the eyepieces for a long time is a burden for an examiner. For this reason, the observation system is provided with an imaging system (camera) branched from the left and right observation optical paths, and a part of the return light is led to the imaging system. As a result, the return light is continuously imaged by the imaging system, so that the examiner can observe the eye to be examined in real time through a display unit.

A device that enables an eye to be examined to be three-dimensionally observable (stereoscopically viewable) is known as the slit lamp microscope including the imaging system and the display unit as described above. For example, each of Patent Literature 1 and Patent Literature 2 discloses a microscope in which return light branched from a pair of left and right observation optical paths is imaged in a stereo mode by an imaging system, and a pair of left and right observation images obtained by this stereo imaging is displayed in a stereoscopically viewable manner on the display unit.

### Citation List

### Patent Literature

{PTL 1} Japanese Patent Application Laid-Open No. 2019-107552
{PTL 2} Japanese Patent Application Laid-Open No. H07-222720

### Summary of Invention

### Technical Problem

When an eye to be examined is observed with a slit lamp microscope, for some types of disease at sites to be observed, it is preferable that a pair of left and right observation images of an eye to be examined obtained by the above-mentioned stereo imaging be displayed in a stereoscopically viewable manner on a display unit. On the other hand, when an anterior eye part of the eye to be examined is simply observed, it is unnecessary to perform the above-mentioned stereo imaging, and it is sufficient only to perform monaural imaging in which only return light branched from any one of a pair of left and right observation optical paths is imaged by an imaging system, and display an observation image obtained by this monaural imaging on the display unit. Therefore, it is desirable to freely perform switching between acquisition of an observation image based on stereo imaging and acquisition of an observation image based on monaural imaging so that an examiner can easily observe an eye to be examined according to observation target and purpose.

Here, when monaural imaging is performed with a slit lamp microscope, it has been conventionally general that, among return lights each branched from a pair of left and right observation optical paths, only the return light from the observation optical path corresponding to one of the left and right eyes of an examiner is subjected to monaural imaging, and an observation image obtained by the monaural imaging is displayed on a display unit. At this time, for example, when the monaural imaging is performed on the return light from the observation optical path corresponding to the examiner's right eye, there is no problem if the examiner's dominant eye is the right eye. However, if the dominant eye is the left eye, there is a risk that a difference in appearance occurs between an image of the eye to be examined observed through the display unit by the examiner and an image of the eye to be examined observed through the eyepiece by the examiner, which makes the examiner feel uncomfortable. For this reason, depending on the dominant eye of the examiner, it is desirable that the acquisition of an observation image obtained by imaging only the return light from the observation optical path on the right eye side and the acquisition of an observation image obtained by imaging only the return light from the observation optical path on the left eye side be freely switched from each other.

The presently disclosed subject matter has been made in view of the above circumstances, and has an object to provide an ophthalmic microscope capable of acquiring an observation image that meets observation target and purpose of an eye to be examined and an examiner's dominant eye.

### Solution to Problem

An ophthalmic microscope for achieving the above object of the presently disclosed subject matter includes: an observation system configured to observe an eye to be examined. the observation system including an objective lens and a pair of observation optical paths; an imaging system capable of stereoscopically imaging respective observation lights of the eye to be examined from the observation optical paths; and a mode switching unit capable of selectively switching among a first mode for acquiring a first image obtained by stereoscopically imaging the observation lights from both the observation optical paths using the imaging system, a second mode for acquiring only a second image obtained by imaging the observation light from one of the observation optical paths using the imaging system, and a third mode for acquiring only a third image obtained by imaging the observation light from the other of the observation optical paths using the imaging system.

According to the ophthalmic microscope, an optimum mode can be freely switched from among the first mode to the third mode according to the observation target and purpose of the eye to be examined and the examiner's dominant eye.

In an ophthalmic microscope according to another aspect of the presently disclosed subject matter, the mode switching unit includes: shutters each provided to be freely insertable into and removable from a corresponding one of the observation optical paths; and a shutter switching mechanism configured to: retract the shutters from both the observation optical paths in the first mode, retract the one of the shutters from one of the observation optical paths and insert the other of the shutters into the other of the observation optical paths in the second mode, and insert the one of the shutters into one of the observation optical paths and retract the other of the shutters from the other of the observation optical paths in the third mode. Accordingly, an optimum mode can be freely switched from among the first mode to the third mode according to the observation target and purpose of the eye to be examined and the examiner's dominant eye.

In an ophthalmic microscope according to another aspect of the presently disclosed subject matter, the imaging system stereoscopically images the observation lights from both the observation optical paths and outputs the first image, the mode switching unit includes a trimming unit configured to trim the first image output from the imaging system, and the trimming unit trims the second image from the first image in the second mode, trims the third image from the first image in the third mode, and is in a standby state in the first mode. Accordingly, an optimum mode can be freely switched from among the first mode to the third mode according to the observation target and purpose of the eye to be examined and the examiner's dominant eye.

In an ophthalmic microscope according to another aspect of the presently disclosed subject matter, the observation system includes binocular eyepieces, the one of the observation optical paths is provided between one of the eyepieces and the objective lens, and the other of the observation optical paths is provided between the other of the eyepieces and the objective lens, wherein the ophthalmic microscope includes a deflection element capable of deflecting parts of the observation lights passing through the one and the other of the observation optical paths out of optical paths of the observation optical paths, and wherein the imaging system is capable of imaging the observation light of the one of the observation optical paths which has been deflected by the deflection element, and the observation light of the other of the observation optical paths which has been deflected by the deflection element.

In an ophthalmic microscope according to another aspect of the presently disclosed subject matter, the imaging system is capable of imaging, by using a common imaging element, both the observation lights each deflected by the deflection element for each of the observation optical paths.

In an ophthalmic microscope according to another aspect of the presently disclosed subject matter, the deflection element is provided so as to straddle both the observation optical paths.

An ophthalmic microscope according to another aspect of the presently disclosed subject matter includes a variable magnification optical system provided between the objective lens and the deflection element for each of the observation optical paths; and an aperture provided between the deflection element and the variable magnification optical system for each of the observation optical paths. Accordingly, observation light which is incident from any one of the observation optical paths to the imaging system is prevented from being mixed with observation light from the other of the observation optical paths.

An ophthalmic microscope according to another aspect of the presently disclosed subject matter includes a display unit configured to display the second image in the second mode, display the third image in the third mode, and display the first image in a stereoscopically viewable manner in the first mode.

### Advantageous Effects of Invention

According to the presently disclosed subject matter, it is possible to acquire an observation image which meets observation target and purpose of an eye to be examined and an examiner's dominant eye.

### Brief Description of Drawings

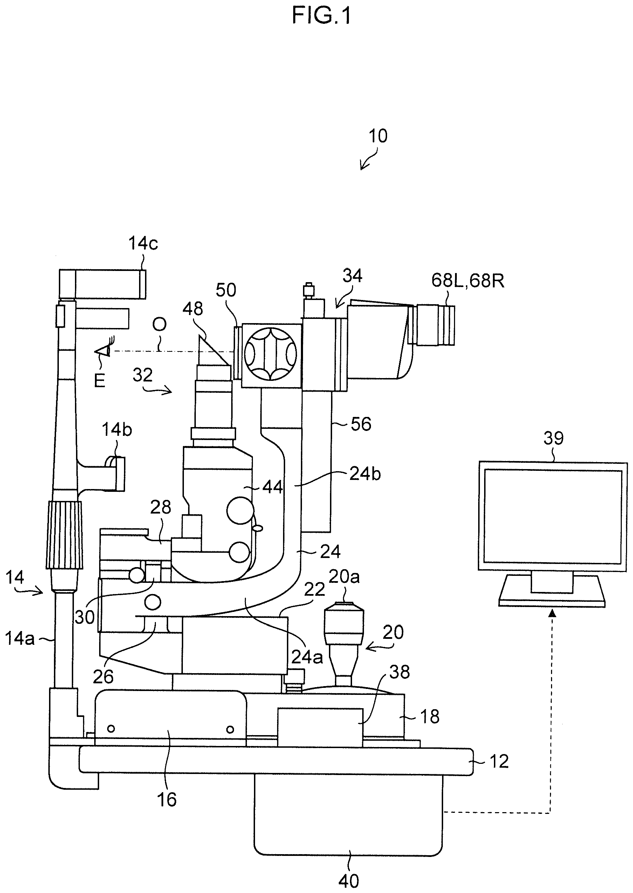

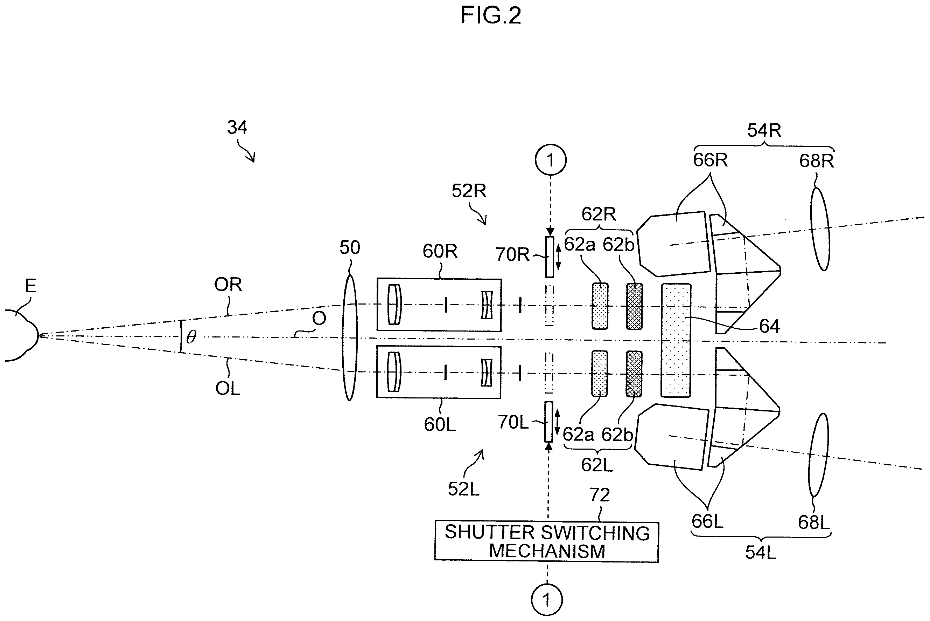

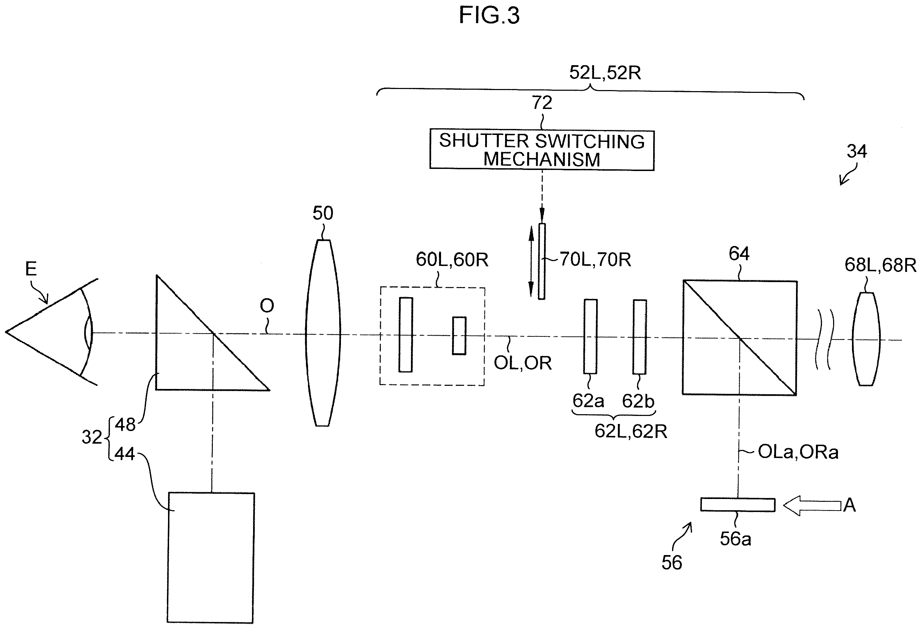

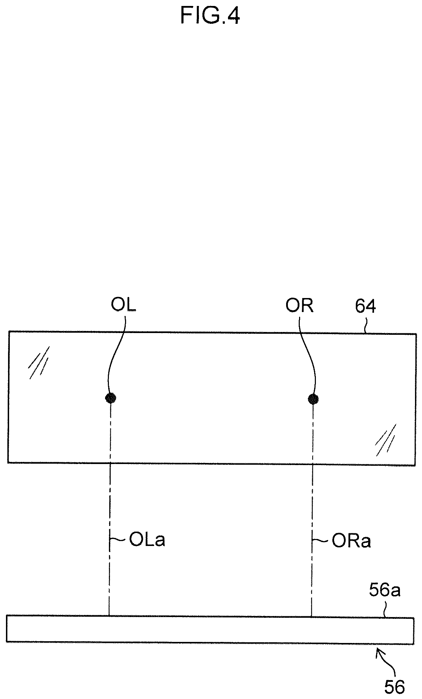

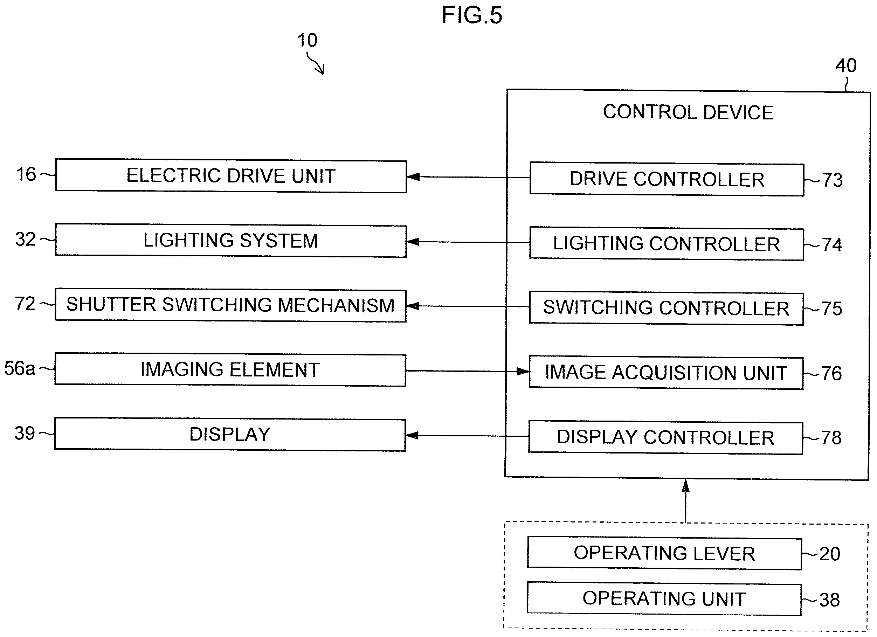

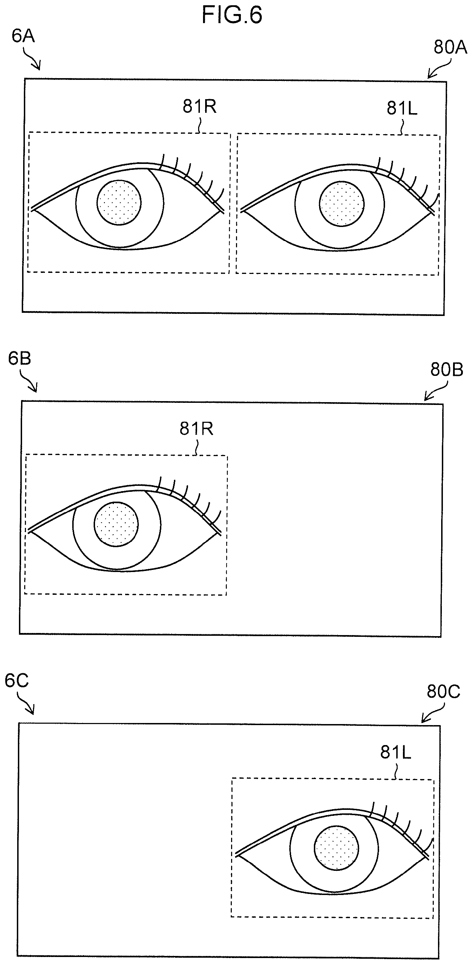

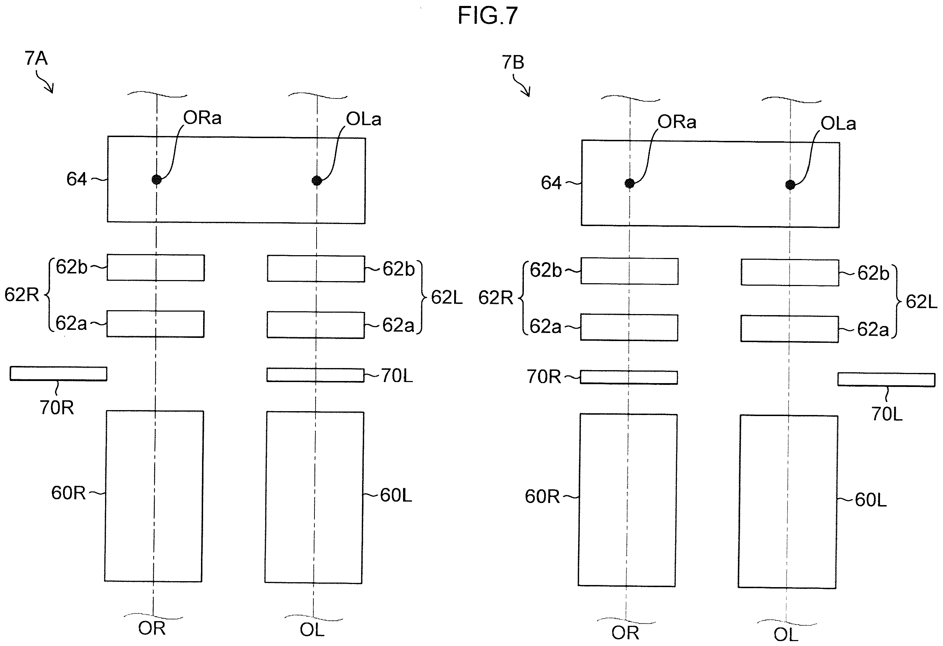

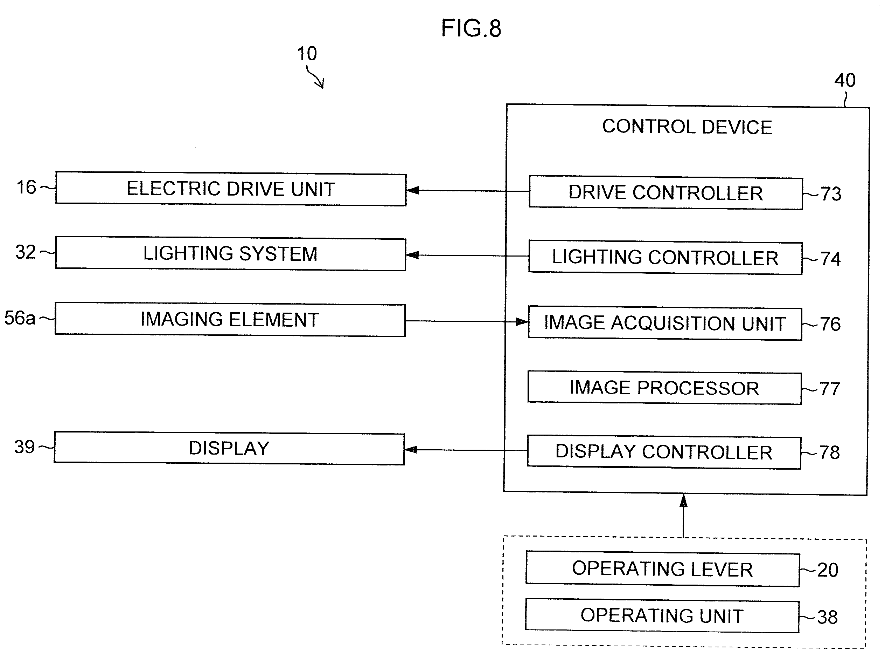

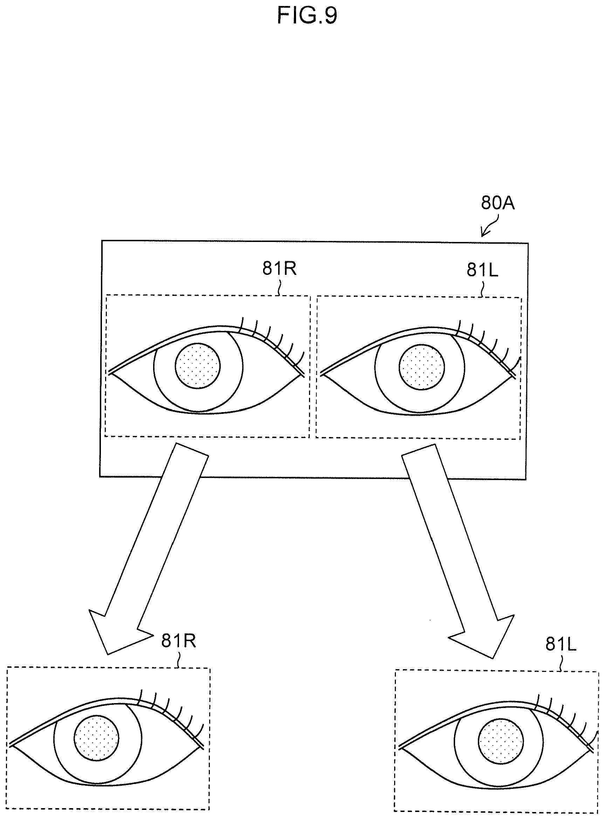

{Figure 1} Figure 1 is a side view of a slit lamp microscope of a first embodiment.
{Figure 2} Figure 2 is an optical layout diagram illustrating the arrangement of an optical system of the microscope as seen from above.
{Figure 3} Figure 3 is an optical layout diagram illustrating the arrangement of the optical system of the microscope as seen from a side.
{Figure 4} Figure 4 is a schematic diagram of an imaging system as seen in an A-direction in Figure 3.
{Figure 5} Figure 5 is a functional block diagram of a control device of the first embodiment.
{Figure 6} Figure 6 is an explanatory diagram illustrating a stereo imaging mode, a right viewpoint image acquisition mode, and a left viewpoint image acquisition mode of the microscope.
{Figure 7} Figure 7 is an explanatory diagram illustrating shutter insertion/removal control by a switching controller in the right viewpoint image acquisition mode and the left viewpoint image acquisition mode.
{Figure 8} Figure 8 is a functional block diagram of a control device for a slit lamp microscope according to a second embodiment.
{Figure 9} Figure 9 is an explanatory diagram for explaining image processing (trimming) by an image processor 77.

### Description of Embodiments

### [First Embodiment]

Figure 1 is a side view of a slit lamp microscope 10 according to a first embodiment. The slit lamp microscope 10 corresponds to an ophthalmic microscope of the presently disclosed subject matter, and includes a base 12, a face supporter 14, an electric drive unit 16, a movable table 18, an operating lever 20, a first supporter 22, a microscope supporting arm 24, a turning shaft 26, a second supporter 28, a turning shaft 30, a lighting system 32, a microscope 34, an operating unit 38, a display unit 39, and a control device 40.

The base 12 is placed on an eye examination table (not illustrated). The face supporter 14 is provided on an upper surface of the base 12 and at a front end portion of a subject (on an eye to be examined E side). Further, the electric drive unit 16 and the operating unit 38 are provided on the upper surface of the base 12, and the movable table 18 is held on the upper surface of the base 12 so as to be movable in a horizontal direction (a front-rear direction and a left-right direction). Here, the front-rear direction includes a frontward direction in which the movable table 18 approaches the subject and a rearward direction in which the movable table 18 moves away from the subject, and the left-right direction is an eye-width direction of the subject.

The face supporter 14 includes a pair of columns 14a which are fixed to the base 12 and extend in a vertical direction, a chin rest 14b which is provided at intermediate portions in the vertical direction of the pair of columns 14a, a forehead rest 14c which is provided at upper end portions in the vertical direction of the pair of columns 14a. The subject places his/her chin on the chin rest 14b and also attaches his/her forehead to the forehead rest 14c, whereby the subject's face is supported by the face supporter 14. As a result, the position of the eye to be examined E is fixed.

The electric drive unit 16 moves the movable table 18 in the horizontal direction (front-rear direction and left-right direction) on the base 12. Further, the operating lever 20 is provided on the upper surface of the movable table 18 and at a rear end portion on the rearward side (examiner side). Still further, the first supporter 22 is provided on the upper surface of the movable table 18 so as to be movable in the vertical direction (movable up and down).

The electric drive unit 16 includes motors (not illustrated) and a drive transmission mechanism (not illustrated) for converting the rotations of the respective motors into driving forces in the horizontal direction and the vertical direction. This electric drive unit 16 moves the movable table 18 in the horizontal direction and also moves the first supporter 22 in the vertical direction, according to the operation of the operating lever 20. Thereby, the position of the first supporter 22 (the lighting system 32 and the microscope 34) with respect to the eye to be examined E can be adjusted.

The operating lever 20 is an operating member for manually moving the first supporter 22 (the lighting system 32 and the microscope 34) in each of the horizontal direction and the vertical direction. For example, by tilting the operating lever 20 in the front-rear direction or in the left-right direction, the electric drive unit 16 moves the movable table 18 in the front-rear direction or in the left-right direction. Further, a turning operation of the operating lever 20 around an axial line causes the electric drive unit 16 to move the first supporter 22 in the vertical direction. A switch 20a to be used for imaging, etc. is provided at the top of the operating lever 20.

The microscope supporting arm 24 is disposed on the first supporter 22. This microscope supporting arm 24 has a horizontal arm portion 24a and a vertical arm portion 24b, and is formed in a substantially L-shape.

An end portion on the frontward side of the horizontal arm portion 24a is horizontally turnably attached onto the first supporter 22 via the turning shaft 26 extending in the vertical direction. Further, the second supporter 28 is horizontally turnably attached onto the horizontal arm portion 24a via the turning shaft 30 located on an extension line of the turning shaft 26.

Here, the horizontal turning of the microscope supporting arm 24 around the turning shaft 26 and the horizontal turning of the second supporter 28 around the turning shaft 30 may be performed manually by the examiner or may be performed electrically using an electric turning mechanism (not illustrated).

The microscope 34 is attached to an upper end portion of the vertical arm portion 24b. Further, the second supporter 28 is provided with the lighting system 32.

The lighting system 32 irradiates the eye to be examined E with slit light. This lighting system 32 includes a slit lamp 44 and a deflection element 48. The slit lamp 44 emits slit light toward the deflection element 48. Here, the configuration of the slit lamp 44 is a known technique, and thus detailed description thereof is omitted.

The deflection element 48 is provided above the slit lamp 44. For example, a prism is used as the deflection element 48, and it deflects the slit light emitted from the slit lamp 44 toward the eye to be examined E. Here, a mirror (reflection mirror) may be used as the deflection element 48 instead of the prism. This causes the eye to be examined E to be irradiated with the slit light.

The lighting system 32 is horizontally turned integrally with the second supporter 28 around the turning shaft 30. As a result, it is possible to adjust the irradiation direction of the slit light to the eye to be examined E.

The lighting system 32 (the slit lamp 44 and the deflection element 48) is not limited to the configuration illustrated in Figure 1, and the shape, structure, and arrangement thereof may be changed as appropriate.

The microscope 34 corresponds to the observation system of the presently disclosed subject matter, and is used to observe the eye to be examined E being illuminated by the lighting system 32. An objective lens 50 is provided at a front end portion on the frontward side (eye to be examined E side) of the microscope 34, and a pair of left and right eyepieces 68L and 68R are provided at a rear end portion on the rearward side (examiner side). Reference character O in Figure 1 designates the optical axis of the objective lens 50.

The microscope 34 is horizontally turned integrally with the microscope supporting arm 24 around the turning shaft 26. As a result, it is possible to adjust the observation direction of the eye to be examined E to be observed by the microscope 34. Further, the microscope 34 is also provided with an imaging system 56 for imaging the eye to be examined E through the optical system of the microscope 34.

The operating unit 38 is provided on the upper surface of the base 12 and at the rear end portion on the rearward side (examiner side). This operating unit 38 is used for turning on and off the slit lamp 44 by the examiner and switching the operation mode of the microscope 34 as described in detail later.

The display unit 39 is a publicly known monitor such as an LCD (Liquid Crystal Display), which is capable of displaying a pair of left and right images obtained by stereo imaging in a stereoscopically viewable manner. An observation image of the eye to be examined E captured by the imaging system 56 is displayed on the display unit 39, and if this observation image is an image obtained by stereo imaging, it is displayed to be stereoscopically viewable. As a result, the examiner can observe the eye to be examined E (including stereoscopic vision) through the display unit 39.

The control device 40 is an arithmetic processing device such as a computer for executing various arithmetic processing and control processing, and is provided, for example, on the bottom surface of the base 12. The location where the control device 40 is provided is not particularly limited. Each part of the slit lamp microscope 10 is connected to this control device 40. The control device 40 centrally controls the operation of each part of the slit lamp microscope 10 based on an operation instruction input to the operating lever 20 and the operating unit 38. For example, the control device 40 performs the position adjustment of the lighting system 32 and the microscope 34 by the electric drive unit 16, the switching of the operation mode of the microscope 34, the control of the imaging system 56. and the display control of the display unit 39.

Figure 2 is an optical layout diagram illustrating the arrangement of the optical system of the microscope 34 as seen from above. Figure 3 is an optical layout diagram illustrating the arrangement of the optical system of the microscope 34 as seen from a side.

As illustrated in Figures 2 and 3, the microscope 34 is a binocular type capable of stereoscopically viewing the eye to be examined E, and includes the objective lens 50, a pair of left and right observation optical paths 52L and 52R (also referred to as a relay optical system), a pair of left and right eyepiece systems 54L and 54R, and the imaging system 56. Here, reference character OL in the figures designates an observation optical axis extending from the objective lens 50 through the observation optical path 52L to the left eye eyepiece system 54L, and reference character OR in the figures designates an observation optical axis extending from the objective lens 50 through the observation optical path 52R to the right eye eyepiece system 54R. The observation optical axes OL and OR intersect each other at a predetermined convergence angle θ at the objective lens 50.

The observation optical path 52L is an optical system (optical path) for leading return light (observation light) from the eye to be examined E irradiated with slit light up to the eyepiece system 54L. A variable magnification unit 60L, an aperture 62L, and a beam splitter 64 are arranged from the objective lens 50 along the observation optical axis OL in the observation optical path 52L.

The observation optical path 52R is an optical system for leading return light up to the eyepiece system 54R. A variable magnification unit 60R, an aperture 62R, and the beam splitter 64 are arranged from the objective lens 50 along the observation optical axis OR in the observation optical path 52R.

The variable magnification units 60L and 60R are publicly known variable magnification optical systems, and are used to change the magnification of an observation image to be observed with the microscope 34.

Each of the apertures 62L and 62R includes, for example, a field stop 62a and an imaging stop 62b, and is provided between the variable magnification unit 60L, 60R and the eyepiece system 54L, 54R (more specifically, the beam splitter 64). Here, the type and number of the apertures 62L and 62R are not particularly limited. By providing the apertures 62L and 62R between the variable magnification units 60L and 60R and the beam splitter 64, return lights that respectively pass from the observation optical paths 52L and 52R through the beam splitter 64 and enter the imaging system 56 can be separated from each other. In other words, the return light which is incident from any one of the observation optical paths 52L and 52R to the imaging system 56 is prevented from being mixed with the return light from the other of the observation optical paths 52L and 52R.

The beam splitter 64 corresponds to the deflection element of the presently disclosed subject matter, and is provided so as to straddle both the observation optical paths 52L and 52R. The beam splitter 64 deflects a part of the return light from each of the observation optical paths 52L and 52R to the imaging system 56 (outside the optical paths of the observation optical paths 52L and 52R). Here, reference character OLa in Figure 3 designates a branch optical axis branched from the observation optical axis OL by the beam splitter 64, and reference character ORa designates a branch optical axis branched from the observation optical axis OR by the beam splitter 64.

The beam splitter 64 also emits the remainder of the return light passing through the observation optical path 52L to the eyepiece system 54L, and also emits the remainder of the return light passing through the observation optical path 52R to the eyepiece system 54R. The beam splitter 64 may be provided individually for each of the observation optical paths 52L and 52R.

The eyepiece system 54L includes a prism unit 66L and an eyepiece 68L. Further, the eyepiece system 54R includes a prism unit 66R and an eyepiece 68R. This also enables the examiner to observe the eye to be examined E by gazing through the eyepieces 68L and 68R.

Figure 4 is a schematic diagram of the imaging system 56 as seen in an A-direction in Figure 3. As illustrated in Figure 4 and Figure 3 described above, the imaging system 56 includes an imaging lens (not illustrated), and a common imaging element 56a for imaging the return light from each of the observation optical paths 52L and 52R that has been deflected by the beam splitter 64.

The imaging element 56a is a CMOS (Complementary Metal Oxide Semiconductor) type or a CCD (Charge Coupled Device) type, and has a rectangular light receiving face which is perpendicular to branch optical axes OLa and ORa and arranged to straddle both the branch optical axes OLa and ORa. As a result, the imaging element 56a can simultaneously image the respective return lights from the observation optical paths 52L and 52R, in other words, can perform stereo imaging. At this time, as described above, since the return lights to be incident to the imaging element 56a are separated from each other by the apertures 62L and 62R provided between the variable magnification units 60L and 60R and the beam splitter 64, an excellent image can be obtained even when the respective return lights from the observation optical paths 52L and 52R are simultaneously imaged by the common imaging element 56a.

In the present embodiment, the respective return lights from the observation optical paths 52L and 52R are allowed to be simultaneously imaged using the common imaging element 56a, but the return light from each of the observation optical paths 52L and 52R may be individually imaged using an individual imaging element 56a.

Returning to Figures 2 and 3, a shutter 70L is provided in the observation optical path 52L so as to be freely insertable into and removable from the observation optical path 52L, and a shutter 70R is provided in the observation optical path 52R so as to be freely insertable into and removable from the observation optical path 52R. The insertion and removal of these shutters 70L and 70R for the observation optical paths 52L and 52R are performed by a shutter switching mechanism 72. The position of the shutter 70L on the observation optical path 52L and the position of the shutter 70R on the observation optical path 52R are not limited to the arrangement illustrated in Figures 2 and 3, and the positions may be changed on the observation optical paths 52L and 52R as appropriate. Further, the shutters 70L and 70R may be provided between the beam splitter 64 and the imaging element 56a.

The shutter switching mechanism 72 functions as a mode switching unit of the presently disclosed subject matter together with the shutters 70L and 70R described above. The shutter switching mechanism 72 is configured by an actuator (not illustrated), and under the control of the control device 40 described later, the shutter switching mechanism 72 individually performs the insertion and removal of the shutter 70L for the observation optical path 52L, and the insertion and removal of the shutter 70R for the observation optical path 52R. This makes it possible to retract the shutters 70L and 70R from both the observation optical paths 52L and 52R, retract the shutter 70L only from the observation optical path 52L, and retract the shutter 70R only from the observation optical path 52R.

Figure 5 is a functional block diagram of the control device 40 of the first embodiment. As illustrated in Figure 5, the functions of the control device 40 are implemented using various processors. The various processors include CPU (Central Processing Unit), GPU (Graphics Processing Unit), ASIC (Application Specific Integrated Circuit), and programmable logic devices [for example, SPLD (Simple Programmable Logic Devices), CPLD (Complex Programmable Logic Device), and FPGA (Field Programmable Gate Arrays)], etc. The various functions of the control device 40 may be implemented by one processor, or may be implemented by processors of the same type or different types.

The control device 40 functions as a drive controller 73, a lighting controller 74, a switching controller 75, an image acquisition unit 76, and a display controller 78 by executing programs read from a storage unit (not illustrated).

The drive controller 73 drives the electric drive unit 16 in response to an input operation on the operating lever 20 to move the lighting system 32 and the microscope 34 forward, backward, leftward, rightward, upward and downward, thereby performing the positional adjustment on the lighting system 32 and the microscope 34 for the eye to be examined E.

The lighting controller 74 sets the emission of slit light from the slit lamp 44 to ON or OFF in response to an on/off operation of the slit lamp 44 (or an on/off operation of a power supply of the slit lamp microscope 10) on the operating unit 38.

The switching controller 75 drives the shutter switching mechanism 72 in response to a switching operation of the operation mode of the microscope 34 on the operating unit 38 to individually control the insertion and removal of the shutter 70L for the observation optical path 52L and the insertion and removal of the shutter 70R for the observation optical path 52R.

The operation mode of the microscope 34 includes a stereo imaging mode (corresponding to a first mode of the presently disclosed subject matter), a right viewpoint image acquisition mode (corresponding to a second mode of the presently disclosed subject matter), and a left viewpoint image acquisition mode (corresponding to a third mode of the presently disclosed subject matter).

Figure 6 is an explanatory diagram illustrating the stereo imaging mode, the right viewpoint image acquisition mode, and the left viewpoint image acquisition mode of the microscope 34. As designated by reference numeral 6A in Figure 6, the stereo imaging mode is a mode to be selected, for example, when a specific disease of the eye to be examined E is observed, and is a mode for acquiring an observation image 80A to be obtained by performing stereo imaging on return lights from the observation optical paths 52L and 52R using the imaging element 56a. This observation image 80A includes a pair of left and right images of the eye to be examined E, which corresponds to a first image of the presently disclosed subject matter, that is, includes a left viewpoint image 81L obtained by imaging the return light from the observation optical path 52L and a right viewpoint image 81R obtained by imaging the return light from the observation optical path 52R.

As designated by reference numeral 6B in Figure 6, the right viewpoint image acquisition mode is a mode to be selected for observation of the anterior eye part of the eye to be examined E by an examiner whose dominant eye is the right eye (in this case, observation of the eye to be examined E that does not require stereoscopic vision), and also a mode for acquiring an observation image 80B obtained by imaging only the return light from the observation optical path 52R using the imaging element 56a. This observation image 80B includes only a right viewpoint image 81R which corresponds to a second image of the presently disclosed subject matter.

As designated by reference numeral 6C in Figure 6, the left viewpoint image acquisition mode is a mode to be selected for observation of the anterior eye part of the eye to be examined E by an examiner whose dominant eye is the left eye, and also a mode for acquiring an observation image 80C obtained by imaging only the return light from the observation optical path 52L using the imaging element 56a. This observation image 80B includes only a left viewpoint image 81L which corresponds to a third image of the presently disclosed subject matter.

Returning to Figure 5, when a switching operation to the stereo imaging mode is performed by the operating unit 38, the switching controller 75 drives the shutter switching mechanism 72 to retract the shutters 70L and 70R from both the observation optical paths 52L and 52R (see Figure 2). As a result, the return lights from both the observation optical paths 52L and 52R enter the light receiving surface of the imaging element 56a, and are imaged by the imaging element 56a, so that the observation image 80A is output from the imaging element 56a.

Figure 7 is an explanatory diagram illustrating the insertion and removal control of the shutters 70L and 70R by the switching controller 75 in the right viewpoint image acquisition mode and the left viewpoint image acquisition mode. As designated by reference numeral 7A in Figure 7, when a switching operation to the right viewpoint image acquisition mode is performed by the operating unit 38, the switching controller 75 drives the shutter switching mechanism 72 to retract the shutter 70R from the observation optical path 52R, and also inserts the shutter 70L into the observation optical path 52L. As a result, only the return light from the observation optical path 52R enters the light receiving surface of the imaging element 56a, and is imaged by the imaging element 56a, so that the observation image 80B is output from the imaging element 56a.

As designated by reference numeral 7B in Figure 7, when a switching operation to the left viewpoint image acquisition mode is performed by the operating unit 38, the switching controller 75 drives the shutter switching mechanism 72 to retract the shutter 70L from the observation optical path 52L, and also inserts the shutter 70R into the observation optical path 52R. As a result, only the return light from the observation optical path 52L enters the light receiving surface of the imaging element 56a, and is imaged by the imaging element 56a, so that the observation image 80C is output from the imaging element 56a.

Returning to Figure 5, the image acquisition unit 76 is connected, by wire or wirelessly, to the imaging element 56a via a communication interface (not illustrated). The image acquisition unit 76 acquires an observation image (any of observation images 80A, 80B, and 80C) from the imaging element 56a in each operation mode of the stereo imaging mode, the right viewpoint image acquisition mode, and the left viewpoint image acquisition mode, and outputs the acquired observation image to the display controller 78.

The display controller 78 controls the display of the display unit 39. In the stereo imaging mode, the display controller 78 extracts a left viewpoint image 81L and a right viewpoint image 81R from the observation image 80A acquired from the image acquisition unit 76, and causes the display unit 39 to display the left viewpoint image 81L and the right viewpoint image 81R so that the images 81L and 81R are stereoscopically viewable. Here, specific display method is a publicly known technique (for example, a lenticular lens method, an active shutter method, etc.), and thus description thereof is omitted.

Further, in the right viewpoint image acquisition mode, the display controller 78 extracts the right viewpoint image 81R from the observation image 80B acquired from the image acquisition unit 76, and causes the display unit 39 to display this right viewpoint image 81R. Further, in the left viewpoint image acquisition mode, the display controller 78 extracts the left viewpoint image 81L from the observation image 80C acquired from the image acquisition unit 76, and causes the display unit 39 to display this left viewpoint image 81L.

As described above, in the present embodiment, provision of the shutters 70L and 70R and the shutter switching mechanism 72 in the microscope 34 enables the microscope 34 to perform the switching between stereo imaging and monaural imaging and also the switching between the observation optical paths 52R and 52L to be used during monaural imaging. As a result, it is possible to perform the switching between stereo imaging and monaural imaging (right viewpoint image acquisition mode, left viewpoint image acquisition mode) in accordance with the observation target and purpose of the eye to be examined E, and also perform the switching between the right viewpoint image acquisition mode and the left viewpoint image acquisition mode according to the examiner's dominant eye during the monaural imaging. As a result, it is possible to acquire an observation image (observation images 80A to 80C) of the eye to be examined E which meets the observation target and purpose of the eye to be examined E and the examiner's dominant eye.

For example, when stereoscopic vision of the eye to be examined E is preferable depending on the type of disease of the eye to be examined E or the like, the observation image 80A (the left viewpoint image 81L and the right viewpoint image 81R) is acquired by switching the microscope 34 to the stereo imaging mode, and the left viewpoint image 81 L and the right viewpoint image 81R are displayed on the display unit 39 so as to be stereoscopically viewable.

Further, when the anterior eye part of the eye to be examined E is observed and the examiner's dominant eye is the right eye, by switching the microscope 34 to the right viewpoint image acquisition mode, only the return light from the observation optical path 52R can be imaged by the imaging element 56a to acquire an observation image 80B, and the right viewpoint image 81R can be displayed on the display unit 39 based on this observation image 80B. Furthermore, when the examiner's dominant eye is the left eye, by switching the microscope 34 to the left viewpoint image acquisition mode, only the return light from the observation optical path 52L can be imaged by the imaging element 56a to acquire an observation image 80C, and the left viewpoint image 81L can be displayed on the display unit 39 based on this observation image 80C. This makes it possible to display an image of the eye to be examined E (the right viewpoint image 81R or the left viewpoint image 81L) corresponding to the examiner's dominant eye on the display unit 39. As a result, the image of the eye to be examined E observed through the display unit 39 and the image of the eye to be examined observed through the eyepieces 68L and 68R appear in the same manner, so that the examiner's discomfort can be reduced.

### [Second Embodiment]

Figure 8 is a functional block diagram of a control device 40 of a slit lamp microscope 10 of a second embodiment. In the first embodiment, the operation mode of a microscope 34 can be selectively switched by controlling the insertion and removal of shutters 70L and 70R by a shutter switching mechanism 72. However, in the second embodiment, the operation mode can be selectively switched without providing the shutters 70L and 70R, etc. in the microscope 34.

As illustrated in Figure 8, the slit lamp microscope 10 of the second embodiment has basically the same configuration as the first embodiment except that the control device 40 functions as an image processor 77 instead of providing the shutters 70L and 70R and the shutter switching mechanism 72. Therefore, the same reference signs are given to the same elements in function or configuration as those in the first embodiment, and the description thereof is omitted.

Since the microscope 34 of the second embodiment does not include the shutters 70L and 70R, an imaging element 36a always images return lights from both observation optical paths 52L and 52R, and an image acquisition unit 76 always acquires an observation image 80A from the imaging element 36a, and outputs the observation image 80A to the image processor 77.

Figure 9 is an explanatory diagram for explaining image processing (trimming) by the image processor 77. As illustrated in Figure 9, the image processor 77 corresponds to a mode switching unit and a trimming unit of the presently disclosed subject matter, and trimming processing is executed on an observation image 80A (a right viewpoint image 81R and a left viewpoint image 81L) input from the image acquisition unit 76 when the operation mode selected by an operating unit 38 is a right viewpoint image acquisition mode or a left viewpoint image acquisition mode.

When the operation mode is switched to the right viewpoint image acquisition mode by the operating unit 38, the image processor 77 trims only the right viewpoint image 81R from the observation image 80A input from the image acquisition unit 76, and outputs this right viewpoint image 81R to a display controller 78. As a result, the display controller 78 displays the right viewpoint image 81R on a display unit 39.

Further, when the operation mode is switched to the right viewpoint image acquisition mode by the operating unit 38, the image processor 77 trims only the left viewpoint image 81L from the observation image 80A input from the image acquisition unit 76, and outputs this left viewpoint image 81L to the display controller 78. As a result, the display controller 78 displays the left viewpoint image 81L on the display unit 39.

Further, when the operation mode is switched to a stereo imaging mode by the operating unit 38, the image processor 77 enters a standby state (operation stopped state) and outputs the observation image 80A to the display controller 78 as it is. As a result, the display controller 78 displays the left viewpoint image 81L and the right viewpoint image 81R on the display unit 39 so that the images 81L and 81R are stereoscopically viewable.

As described above, in the second embodiment, since the operation mode can be switched through software processing by the image processor 77, the shutters 70L and 70R, etc. as in the first embodiment are not required, and the manufacturing cost of the slit lamp microscope 10 can be reduced.

### [Others]

In each of the above embodiments, the operation mode is switched by the shutters 70L and 70R or the image processor 77, but the operation mode may be switched by controlling the operation of the imaging element 56a.

For example, the light receiving surface of the imaging element 56a is divided into a first light receiving region that receives the return light from the observation optical path 52R and a second light receiving region that receives the return light from the observation optical path 52L. An imaging controller (mode switching unit) (not illustrated) individually controls imaging/stopping of imaging in each of the first light receiving region and the second light receiving region of the imaging element 56a. Examples of this control include readout control (CMOS type) using an XY address method, partial electronic shutter control, etc. for the imaging element 56a. As a result, it is possible to switch the operation mode without providing the shutters 70L and 70R, etc. as in the second embodiment, so that the same effects as in the second embodiment can be obtained.

In the first embodiment described above, the operation mode of the microscope 34 can be selectively switched by controlling the insertion and removal of the shutters 70L and 70R by the shutter switching mechanism 72. However, when the beam splitter 64 is provided individually for each of the observation optical paths 52L and 52R, the operation mode may be switched by controlling the insertion and removal of the beam splitter 64 for each of the observation optical paths 52L and 52R.

In the above embodiments, the Zeiss type (Littman type) slit lamp microscope 10 in which the lighting system 32 is provided below the deflection element 48 has been described as an example. However, the presently disclosed subject matter can also be applied to a Haag type (Goldmann type) slit lamp microscope 10 in which the lighting system 32 is provided above the deflection element 48.

Each of the above embodiments has been described using the slit lamp microscope 10 as an example, however, the presently disclosed subject matter can be applied to various ophthalmic microscopes such as a surgical microscope.

### Reference Signs List

10: slit lamp microscope, 12: base, 14: face supporter, 14a: column, 14b: chin rest. 14c: forehead rest, 16: electric drive unit, 18: movable table, 20: operating lever, 20a: switch, 22: first supporter, 24: microscope supporting arm, 24a: horizontal arm portion, 24b: vertical arm portion, 26: turning shaft, 28: second supporter, 30: turning shaft, 32: lighting system, 34: microscope, 36a: imaging element, 38: operating unit, 39: display unit, 40: control device, 44: slit lamp, 48: deflection element, 50: objective lens, 52L, 52R: observation optical path, 54L, 54R: eyepiece system, 56: imaging system, 56a: imaging element, 60L, 60R: variable magnification unit, 62L, 62R: aperture, 62a: field stop, 62b: imaging stop, 64: beam splitter, 66L, 66R: prism unit, 68L, 68R: eyepiece, 70L, 70R: shutter, 72: shutter switching mechanism, 73: drive controller, 74: lighting controller, 75: switching controller, 76: image acquisition unit, 77: image processor, 78: display controller, 80A, 80B, 80C: observation image, 81L: left viewpoint image, 81R: right viewpoint image, E: eye to be examined, OL, OR: observation optical axis, OLa, ORa: branch optical axis, Θ: convergence angle

## Claims

1. An ophthalmic microscope comprising:
an observation system configured to observe an eye to be examined, the observation system including an objective lens and a pair of observation optical paths;
an imaging system capable of stereoscopically imaging respective observation lights of the eye to be examined from the observation optical paths; and
a mode switching unit capable of selectively switching among a first mode for acquiring a first image obtained by stereoscopically imaging the observation lights from both the observation optical paths using the imaging system, a second mode for acquiring only a second image obtained by imaging the observation light from one of the observation optical paths using the imaging system, and a third mode for acquiring only a third image obtained by imaging the observation light from the other of the observation optical paths using the imaging system.

2. The ophthalmic microscope according to claim 1, wherein the mode switching unit comprises:
shutters each provided to be freely insertable into and removable from a corresponding one of the observation optical paths; and
a shutter switching mechanism configured to: retract the shutters from both the observation optical paths in the first mode,
retract one of the shutters from the one of the observation optical paths and insert the other of the shutters into the other of the observation optical paths in the second mode, and
insert the one of the shutters into the one of the observation optical paths and retract the other of the shutters from the other of the observation optical paths in the third mode.

3. The ophthalmic microscope according to claim 1, wherein the imaging system stereoscopically images the observation lights from both the observation optical paths and outputs the first image,
the mode switching unit includes a trimming unit configured to trim the first image output from the imaging system, and
the trimming unit trims the second image from the first image in the second mode, trims the third image from the first image in the third mode, and is in a standby state in the first mode.

4. The ophthalmic microscope according to any one of claims 1 to 3, wherein the observation system includes binocular eyepieces, the one of the observation optical paths is provided between one of the eyepieces and the objective lens, and the other of the observation optical paths is provided between the other of the eyepieces and the objective lens,
the ophthalmic microscope comprises a deflection element capable of deflecting parts of the observation lights passing through the one and the other of the observation optical paths out of optical paths of the observation optical paths, and
the imaging system is capable of imaging the observation light of the one of the observation optical paths that has been deflected by the deflection element, and the observation light of the other of the observation optical paths that has been deflected by the deflection element.

5. The ophthalmic microscope according to claim 4, wherein the imaging system is capable of imaging, by using a common imaging element, both the observation lights each deflected by the deflection element for each of the observation optical paths.

6. The ophthalmic microscope according to claim 4 or 5, wherein the deflection element is provided so as to straddle both the observation optical paths.

7. The ophthalmic microscope according to any one of claims 4 to 6, comprising:
a variable magnification optical system provided between the objective lens and the deflection element for each of the observation optical paths; and
an aperture provided between the deflection element and the variable magnification optical system for each of the observation optical paths.

8. The ophthalmic microscope according to any one of claims 1 to 7, comprising a display unit configured to display the second image in the second mode, display the third image in the third mode, and display the first image in a stereoscopically viewable manner in the first mode.
